# FASCICULE DE BREVET EUROPEEN

(11) **EP 1 232 249 B1**
(45) Date de publication et mention de la délivrance du brevet: **07.07.2004**
(21) Numéro de dépôt: 00988869.4
(22) Date de dépôt: 22.11.2000
(51) Int. Cl.: C12N 7/00, A61K 39/155, A61K 39/295

(54) **PNEUMOVIRUS DU CANARD ET VACCINS CORRESPONDANTS**
ENTEPNEUMOVIRUS UND ENTSPRECHENDES IMPFSTOFF
DUCK PNEUMOVIRUS AND CORRESPONDING VACCINES

(30) Priorité: 26.11.1999 FR 9914952
(43) Date de publication de la demande: 21.08.2002
(73) Titulaire: MERIAL, 69002 Lyon (FR)
(72) Inventeur: GOUTEBROZE, Sylvain, Gabriel, F-69007 Lyon (FR)
(74) Mandataire: Colombet, Alain André
(86) Numéro de dépôt international: PCT/FR2000/003255
(87) Numéro de publication internationale: WO 2001/038497

(56) Documents cités:
- EP-A- 0 263 048
- EP-A- 0 292 210

## Description

La présente invention concerne une nouvelle souche de pneumovirus, des vaccins vivants atténués, des vaccins inactivés, des procédés pour vacciner les oiseaux et en particulier les canards contre un pneumovirus, des procédés pour diagnostiquer l'infection par un pneumovirus.

L'infection de la poule et de la dinde par des pneumovirus aviaires (ou APV pour avian pneumovirus) est connue. Les pneumovirus provoquent chez ces espèces des syndromes respiratoires graves touchant le tractus respiratoire supérieur, des pertes de poids, voire la mort de ces animaux. Cette pneumovirose est également dénommée rhinotrachéïte de la dinde (ou turkey rhinotracheitis - TRT) et syndrome de la grosse tête du poulet (ou swollen head syndrome - SHS), ce dernier étant également caractérisé par l'apparition d'oedèmes sous-cutanés sur la tête de l'animal. Les pneumovirus aviaires peuvent également provoquer des chutes de ponte chez les pondeuses. Les conséquences économiques de ces maladies sont importantes.

Il s'agit du même virus nommé virus de la rhinotrachéïte de la dinde (ou TRTV) qui provoque ces deux maladies.

Le genre des *Pneumovirus* appartient à la famille des *Paramyxoviridae,* famille qui comprend également les genres *Paramyxovirus, Morbillivirus* et *Rubulavirus.* Les *Pneumovirus* sont des virus à ARN simple brin non segmenté de polarité négative. Ils sont enveloppés et leur capside est hélicoïdale. Contrairement aux autres *Paramyxoviridae* (notamment le virus de la maladie de Newcastle ou NDV) *les Pneumovirus* ne possèdent ni hémagglutinine ni neuraminidase (Alexander D. J., Vet. Microbiol., 1990, 23, 103-114).

Des pneumovirus aviaires ont été isolés du poulet ou de la dinde en France, Grande-Bretagne, Italie, Espagne, Hongrie, Allemagne, Pays-Bas, Grèce, Brésil, Mexique, Maroc, Afrique du Sud, Taïwan et lsraël. Un virus antigéniquement et génétiquement différent des virus précédemment connus a également été isolé chez la dinde aux Etats-Unis au début de l'année 1997. Ce virus est souvent dénommé virus Colorado (Seal B. S., Virus Research, 1998, **58**, 45-52 ; Ali *et* al., Avian Disease, 1999, **43**, 600-603).

Les différents isolats de ces pneumovirus aviaires montrent une diversité génétique et antigénique qui peut permettre une classification des souches en au moins deux sous-groupes dénommés A et B (Juhasz *et al.,* J. Gen. Virol., 1994, **75**, 2873-2880). Cet article cite quelques souches de pneumovirus aviaires, notamment les souches APV 2119 (du sous-groupe B, provenant d'Italie), 657/4 (du sous-groupe B, provenant de Hongrie), 872S (du sous-groupe B, provenant d'Espagne), CVL 14/1 (du sous-groupe A, provenant du Royaume-Uni).

La souche APV Colorado n'appartient ni au sous-groupe A ni au sous-groupe B (Seal B. S., Virus Research, 1998, **58**, 45-52).

La vaccination contre des pneumovirus aviaires a permis la prévention de la maladie chez la dinde et le poulet. Des vaccins vivants et inactivés sont disponibles. Les souches vaccinales peuvent être du sous-groupe A ou du sous-groupe B et ont été isolées de la dinde ou du poulet. Les vaccins vivants sont atténués sur culture de cellules. Les vaccins vivants sont généralement utilisés dans le jeune âge chez les oiseaux de chair et les reproducteurs. Les vaccins inactivés sont adjuvés et sont indiqués en vaccination de rappel chez les oiseaux reproducteurs ou chez les pondeuses.

Jusqu'à présent, seuls le poulet et la dinde ont été décrits comme hôtes naturels des pneumovirus aviaires. Des expériences d'infections expérimentales avec un pneumovirus aviaire d'origine dinde (souche CVL 14/1, du sous-groupe A décrite dans Wyeth *et al.,* Vet. Rec., 1986, **119**, 139) ont montré la sensibilité à la maladie et une réponse en anticorps chez la dinde, le poulet et le faisan. Les poulets et les dindes montrent des symptômes d'infections des voies respiratoires supérieures (écoulement nasal, éternuement ...) et les faisans de légères conjonctivites.

Dans ces expériences, le pigeon, l'oie et le canard commun (*Anas platyrhynchos*) sont apparus réfractaires au pneumovirus de la dinde (Cough *et al.,* Vet Rec 1988, **123**, 58-59).

La déposante a mis en évidence un nouveau pneumovirus aviaire dont l'hôte privilégié est le canard.

Ce virus a été isolé en France à partir de prélèvements d'organes provenant d'un troupeau de canes communes reproductrices de type Pékin (*Anas platyrhynchos*). Les animaux de ce troupeau présentaient des symptômes de chute de ponte et de mortalité.

Un broyat d'organes a été inoculé sur des cultures de cellules d'embryons de poulets (CEP) exempts d'organismes pathogènes spécifiés. Au 10ème passage sur CEP, un pneumovirus a été identifié par immunofluorescence avec un sérum dirigé contre l'APV souche Colorado. Les tests d'immunofluorescence réalisés avec des sérums spécifiques d'autres virus du canard ou de la poule sont restés négatifs (parvovirus du canard, virus de la peste du canard, réovirus du canard, adénovirus aviaire du groupe 1, virus de la bronchite infectieuse, virus de la maladie de Newcastle, adénovirus du syndrome de chute de ponte 76, virus de l'influenza aviaire, paramyxovirus de type 3, virus de la maladie de Gumboro, virus de l'encéphalomyélite aviaire). Un test d'immunofluorescence réalisé avec des antisérums spécifiques de différentes souches de pneumovirus aviaires de sous-groupe A et de sous-groupe B s'est avéré également négatif. Par ailleurs le virus isolé par la déposante n'est pas hémagglutinant.

Un paramyxovirus a été visualisé par microscopie électronique dans les cultures de CEP et de cellules Véro infectées par le virus.
Ces éléments indiquent qu'un nouveau pneumovirus aviaire infectant le canard a été isolé. Ce virus est antigéniquement différent des autres pneumovirus aviaires des sous-groupes A et B, de la dinde et du poulet précédemment connus et se distingue de la souche APV Colorado par sa spécificité d'hôte.

Le virus a été isolé et cultivé sur culture CEP, puis il a été adapté sur culture de cellules Véro. Les passages sur culture de cellules Véro peuvent être utilisés pour atténuer le virus.

La présente invention a pour objet une culture de ce nouveau virus (nommé C990427) telle que déposée le 25/11/99 auprès de la Collection Nationale de Cultures de Microorganismes (ou CNCM) de l'Institut Pasteur, Paris, France, sous le numéro d'accès l-2353.

La présente invention a aussi pour objet les préparations immunogènes et les vaccins contre la pneumovirose aviaire comprenant, au moins un antigène ou immunogène de la souche APV C990427 dans un véhicule ou excipient acceptable au plan vétérinaire, et éventuellement en présence d'un adjuvant.

La notion de préparation immunogène recouvre ici toute préparation susceptible, une fois administrée aux oiseaux, d'induire une réponse immunitaire dirigée contre le pathogène aviaire considéré. Par vaccin, on entend une préparation capable d'induire une protection efficace. Les vaccins selon l'invention permettent de prévenir et/ou traiter l'infection.

Comme les autres pneumovirus aviaires déjà connus, ce virus peut être utilisé pour immuniser ou vacciner les oiseaux, notamment gallinacés, e.g., dindes, poules et poulets, et plus particulièrement les canards, notamment les canards communs (*Anas platyrhynchos*) et les canards de Barbarie (*Cairina moschata*), contre l'infection causée par les pneumovirus aviaires et en particulier contre l'infection causée par les pneumovirus du canard.

Suivant une première modalité, l'invention a pour objet une préparation immunogène vivante atténuée ou un vaccin vivant atténué.

Le nouveau virus peut être atténué par les méthodes usuelles connues de l'homme de l'art, en particulier par de 10 à 150 passages sur culture de cellules primaires, par exemple sur culture de CEP, ou sur cellules de lignée, par exemple sur cellules Véro, ou sur oeufs embryonnés. On multiplie le nombre de passages jusqu'à l'absence de symptômes significatifs chez les animaux.

Pour produire le virus vaccinal, le virus atténué peut ensuite être multiplié sur cellules primaires, par exemple CEP, ou cellules de lignée, par exemple sur cellules Véro, ou sur oeufs embryonnés. La préparation immunogène ou le vaccin incorporant une quantité adéquate de virus ainsi multipliés peut être congelé ou lyophilisé avec un stabilisateur, notamment le SPGA (EP-B1-0008255). Chaque dose vaccinale peut contenir de 10² à 10⁶ doses infectieuses 50% sur culture de cellules (DICC50) par dose, et de préférence de 10² à 10⁴.

La préparation immunogène atténuée ou le vaccin atténué selon l'invention peut également être mélangé à d'autres valences virales ou bactériennes pour constituer des préparations immunogènes multivalentes ou des vaccins multivalents. Les valences virales ou bactériennes sont choisies parmi les pathogènes aviaires caractéristiques de l'espèce aviaire à vacciner. Elles sont notamment choisies parmi le groupe comprenant le virus de la maladie de Newcastle (NDV), le virus de la bronchite infectieuse (IBV), le virus de la maladie de Gumboro (IBDV), le parvovirus du canard, et les autres pneumovirus aviaires dont TRTV. Ces autres valences peuvent être notamment des valences atténuées classiques ou des valences recombinées.

Les préparations immunogènes atténuées et vaccins atténués peuvent être administrés aux oiseaux individuellement par inoculation soit *in ovo* notamment entre environ 4 à 1 jour(s) avant l'éclosion et de préférence 3 jours avant éclosion, soit par goutte oculaire, par trempage du bec, ou par injection intramusculaire ou sous-cutanée.

Les préparations immunogènes atténuées et vaccins atténués peuvent être administrés collectivement par eau de boisson ou nébulisation. La préparation immunogène ou le vaccin peut être administré après reprise dans un diluant adjuvé, par exemple avec un adjuvant aqueux tel que l'hydroxyde d'alumine.

Suivant une deuxième modalité, l'invention a pour objet une préparation immunogène inactivée ou un vaccin inactivé.

Le virus peut être multiplié sur cellules primaires, par exemple CEP, ou cellules de lignée, par exemple sur cellules Véro, ou sur oeufs embryonnés. Avant ou après inactivation, le virus ainsi produit peut être clarifié notamment par filtration ou par centrifugation, et/ou peut être concentré notamment par ultrafiltration ou précipitation, et/ou peut être purifié notamment par précipitation sélective ou par chromatographie. L'inactivation peut être réalisée par les méthodes usuelles connues de l'homme de l'art, en particulier par un traitement chimique (e.g. formaldéhyde, (β-propiolactone, éthylèneimine, éthylèneimine binaire (BEI)) et/ou thermique. Le virus inactivé peut ensuite être mélangé à un adjuvant, de préférence aqueux, par exemple à base d'hydroxyde d'alumine, ou être formulé sous forme d'émulsion, notamment émulsion eau-dans-huile, composée d'huile minérale ou d'huile métabolisable et d'un ou plusieurs tensioactifs non ioniques. A titre d'exemple, l'émulsion eau-dans-l'huile comprend du polysorbate 80 (e.g. Tween® 80) dans la phase aqueuse, et de l'huile minérale (e.g. Drakeol® 6-VR) et du monooléate de sorbitan (e.g. Span® 80) dans la phase huileuse (Stone *et al.*, Avian Dis., 1978, **22**, 666-674).

Chaque dose vaccinale peut contenir l'équivalent de 10³ à 10⁸ DiCC50 par dose, et de préférence de 10⁵ à 10⁷ avant inactivation. La quantité d'antigène par dose peut également être déterminée par d'autres méthodes mettant en oeuvre des réactions antigènes/anticorps, par exemple par la méthode ELISA.

L'antigène inactivé peut également être mélangé à d'autres valences virales ou bactériennes pour constituer des préparations immunogènes multivatentes ou des vaccins multivalents. Les valences virales ou bactériennes sont choisies parmi les pathogènes aviaires caractéristiques de l'espèce aviaire à vacciner. Elles sont notamment choisies parmi le groupe comprenant le virus de la maladie de Newcastle (NDV), le virus de la bronchite infectieuse (IBV), le virus de la maladie de Gumboro (IBDV), l'adénovirus du syndrome de chute de ponte, le parvovirus du canard, le paramyxovirus de type 3, les autres pneumovirus aviaires dont TRTV. Ces valences peuvent être notamment des valences inactivées classiques, des valences recombinées, des valences à base de sous-unités.

De préférence, les préparations immunogènes inactivées et vaccins inactivés sont administrés aux oiseaux par injection intramusculaire ou sous-cutanée. L'antigène inactivé peut aussi être constitué de, ou comprendre des antigènes fractionnés ou sous-unités.

La présente invention a encore pour objet des compositions comprenant le nouveau pneumovirus C990427 sous forme vivante atténuée ou sous forme inactivée, éventuellement un véhicule ou excipient acceptable sur le plan vétérinaire, et éventuellement un adjuvant.

Les teneurs en C990427 et le choix des adjuvants sont comme décrit plus haut.

La présente invention a encore pour objet des compositions comprenant des antigènes de C990427 fractionnés ou des sous-unités, éventuellement un véhicule ou excipient acceptable sur le plan vétérinaire, et éventuellement un adjuvant.

L'invention a également pour objet des méthodes d'immunisation ou de vaccination des oiseaux, notamment des canards et gallinacés tels que poules, poulets et dindes, contre les maladies dues aux pneumovirus aviaires et en particulier contre les maladies dues aux pneumovirus du canard, méthodes dans lesquelles on administre à l'animal une préparation immunogène ou un vaccin selon l'invention.

Les programmes d'immunisation ou de vaccination des canards de chair peuvent notamment comprendre une ou deux administration(s) sur des animaux âgés d'environ 1 à environ 3 semaines avec un vaccin atténué, par voie intramusculaire ou sous-cutanée, de préférence avec un adjuvant, notamment un adjuvant aqueux tel que l'hydroxyde d'alumine, ou par voie oculaire, de préférence sans adjuvant. Une administration de rappel peut avoir lieu entre 2 et 4 semaines après la première administration. De préférence, lorsque les canards sont destinés à la reproduction on leur administre en plus, vers l'âge de 10 semaines une nouvelle dose de vaccin vivant et, avant la période de ponte, un vaccin inactivé adjuvé sous forme d'émulsion par voie intramusculaire ou par voie sous-cutanée.

Pour les autres espèces aviaires de chair, notamment les dindes et les poulets, on réalise de préférence une ou deux administration(s) chez le jeune d'un vaccin vivant atténué, de préférence non adjuvé, dans l'eau de boisson ou par nébulisation. La première administration peut être réalisée sur des animaux âgés d'environ 14 jours. Une administration de rappel peut avoir lieu entre 2 et 4 semaines après la première administration.

De préférence, lorsque les autres espèces aviaires, notamment les dindes et les poules, sont destinées à la reproduction ou à la ponte, on leur administre en plus, avant la période de ponte, un vaccin inactivé adjuvé, de préférence sous forme d'émulsion, par voie intramusculaire ou par voie sous-cutanée.

L'homme de l'art possède les compétences nécessaires pour définir précisément le nombre d'injection et les doses de chaque vaccin à utiliser pour chaque protocole de vaccination.

Les volumes de doses peuvent être notamment compris entre 0,1 et 0,8 ml, de préférence de l'ordre de 0,3 à 0,5 ml.

La présente invention a encore pour objet l'utilisation du pneumovirus C990427 pour la réalisation d'une préparation immunogène ou d'un vaccin destiné à être administré à un oiseau selon l'invention, notamment un canard, ladite préparation ou vaccin comprenant en outre un véhicule ou excipient acceptable sur le plan vétérinaire, et éventuellement un adjuvant.

La présente invention a également pour objet l'utilisation d'un antigène ou immunogène du pneumovirus C990427, pour la préparation d'une préparation immunogène ou d'un vaccin destiné à être administré à un oiseau selon l'invention, notamment un canard, ladite préparation ou vaccin comprenant en outre un véhicule ou excipient acceptable sur le plan vétérinaire, et éventuellement un adjuvant.

L'invention vise également une telle utilisation dans le cadre de la réalisation d'une préparation immunogène associée ou d'un vaccin associé, ou dans le cadre d'un programme de vaccinations associées.

L'invention concerne également les réactifs utilisés pour le diagnostic de l'infection par le pneumovirus aviaire C990427.

Ce virus peut être utilisé d'une part comme antigène brut, purifié ou sous forme sous-unité, d'autre part sous forme brute, purifiée ou sous-unité pour immuniser des animaux en vue de l'obtention d'anticorps polyclonaux et monoclonaux. Les antigènes et les anticorps sont utilisables dans la méthode de diagnostic selon l'invention et pour l'élaboration de kits de diagnostic, notamment de type ELISA.

L'invention va être maintenant décrite plus en détail à l'aide de modes de réalisation pris à titre d'exemples non limitatifs.

### EXEMPLES

### Exemple 1 : Isolement du virus

Des organes (foie, coeur, oviducte, trachée et rate en mélange d'une part, intestins d'autre part) ont été prélevés sur des canes communes reproductrices de type Pékin (*Anas platyrhynchos*) présentant des symptômes de chute de ponte et de mortalité. Les deux lots d'organes ont été séparément broyés en tampon phosphate (PBS) additionné d'antibiotique (1/10000 de gentamycine), congelés, décongelés, puis clarifiés par centrifugation. Le surnageant de centrifugation a été dilué à nouveau afin d'obtenir une dilution finale des organes de 1/10ème environ (PN) pour le mélange foie, coeur, oviducte, trachée et rate, et de 1150ème environ (PN) pour l'intestin. Les broyats ont été filtrés (0,22 µm).

Une culture de cellules primaires d'embryons de poulets EOPS - exempts d'organismes pathogènes - (CEP1) a été préparée par inoculation de 3×10⁶ cellules par flacon de 25 cm² en milieu F10-199, pH 6,9 à 7,1, additionné de 5% de sérum de veau foetal (SVF). Les cellules ont été incubées à 38°C avec 5% de CO₂.

La composition du milieu F10-199 est la suivante : 60 ml de F10 (10 fois concentré), 40 ml de 199 Hanks (10 fois concentré), 40 ml de TPB, 20 ml de bicarbonate 5,6%, 1 ml de vitamines, eau qsp 1000ml.

Après 24h de culture, le milieu a été rejeté et un mélange à parts égales des deux broyats a été inoculé sur les cellules CEP1 à raison de 0,8 ml par flacon de 25 cm². Après 30 min de contact à 38°C, les cellules ont été rincées avec du milieu de culture. Du milieu de culture additionné de 1 % de SVF a été ensuite ajouté et les cultures ont été remises en incubation comme précédemment (1er passage).

Après trois jours de culture, les cellules ont été traitées à la pronase et 3x10⁶ cellules ont été réinoculées par flacon de 25 cm² en milieu F10-199 additionné de 3% de SVF, afin d'obtenir une culture de CEP secondaires (CEP2). Les cultures ont été remises en incubation comme précédemment pendant sept jours (2ème passage), puis les cultures ont été congelées, décongelées et la suspension ainsi obtenue a été clarifiée par centrifugation.

1 ml du surnageant a été inoculé sur un nouveau tapis de CEP1 et la culture a été poursuivie comme précédemment (3ème passage).

On a ensuite effectué des passages supplémentaires, alternativement sur CEP2 et sur CEP1.

Au 4ème passage, quelques amas de cellules arrondies et réfringentes ont été observés après quatre jours de culture. A partir du 5ème passage, des plages de cellules arrondies et réfringentes et des syncitia ont été observés. A partir du 10ème passage, un effet cytopathique (ECP) généralisé a été observé après 3 jours de culture.

### Exemple 2 : Adaptation du virus sur cellules Véro

Une culture du virus au 11^{éme} passage sur CEP préparée à l'exemple 1 a été congelée, décongelée et clarifiée par centrifugation.

Une culture de cellules Véro a été préparée par inoculation de 10⁶ cellules par flacon de 25 cm² en milieu Eagle modifié (MEM), pH 6,9 à 7,1, additionné de 5% de SVF. Les cellules ont été incubées à 38°C avec 5% de CO₂.

La composition du milieu MEM est la suivante : 200ml de MEM sels de Earle (5 fois concentré), 0,1 ml de biotine 1%, 10 ml de glutamine 200 mM, 1,5 ml de rouge de phénol, 10 ml de glucose 10%, 30 ml de bicarbonate 5,6%, eau qsp 1000 ml.

Après 24h de culture, le milieu a été rejeté et le virus provenant du 11^{ème} passage sur CEP a été inoculé à raison de 1 ml du surnageant de centrifugation décrit ci-dessus par flacon de 25 cm². Les cellules ont été remises en incubation comme précédemment. Après 6 jours de culture, des amas de cellules arrondies et réfringentes, des syncitia ont été observés et des plages de lyse ont commencé à apparaître sur le tapis cellulaire.

Les passages suivants sur cellules Véro ont été réalisés par congélation, décongélation et clarification des cultures, puis inoculation du surnageant à une nouvelle culture de cellules Véro à raison de 0,5 ml de surnageant par flacon de 25 cm². Les cellules ont été remises en incubation comme précédemment.

Chaque culture a été récoltée lorsque l'ECP fut généralisé, environ 3 à 6 jours après le début de la culture.

La suspension virale peut être conservée à une température inférieure ou égale à -40°C avant son utilisation ultérieure.

### Exemple 3 : Caractérisation du virus par immunoflorescence

Le virus isolé selon l'exemple 1 a été identifié par immunofluorescence sur culture de cellules. Des cultures de cellules Véro en microplaques de 96 cupules ont été préparées à raison de 1,8x10⁴ cellules sous 0,2 ml de milieu MEM, pH 6,9 à 7,1, additionné de 3% de SVF par cupule.

Après 24h d'incubation à 38°C avec 5% de CO₂, le milieu a été rejeté et le virus a été inoculé à raison d'environ 200 DICC50 sous 0,2 ml de milieu MEM, pH 6,9 à 7,1, additionné de 1% de SVF. Les plaques ont été incubées comme précédemment pendant de 48 à 72h. Lorsque des plages de lyse ont commencé à apparaître sur le tapis cellulaire, les cultures ont été rincées par un lavage avec du PBS. Une solution d'acétone (100 ml d'acétone pure + 15 ml d'eau distillée) a été ajoutée dans toutes les cupules pour fixer les cellules. Les plaques ont été placées 20 minutes à -20°C. L'acétone a ensuite été rejetée et les plaques ont été séchées. Les plaques ainsi fixées peuvent être conservées à -20°C.

Les sérums monospécifiques du pneumovirus aviaire du sous-groupe A, du pneumovirus aviaire du sous-groupe B, du pneumovirus aviaire de la souche Colorado, du parvovirus du canard, du virus de la peste du canard, du réovirus aviaire, de l'adénovirus aviaire du groupe 1, du virus de la bronchite infectieuse, du virus de la maladie de Newcastle, de l'adénovirus du syndrome de chute de ponte 76, du virus de l'influenza aviaire, du paramyxovirus de type 3, du virus de la maladie de Gumboro et du virus de l'encéphalomyélite aviaire ont été dilués séparément au 1/20 en eau physiologique. Chaque sérum a été mis séparément en contact avec les cellules Véro infectées à raison de 100 µl de sérum dilué par cupule.

Les plaques ont été placées dans une étuve à 38°C pendant 60 minutes, puis les sérums ont été éliminés et les plaques rincées 3 fois avec de l'eau physiologique et 3 fois avec de l'eau déminéralisée.

Un conjugué fluorescent anti-poulet du commerce a été dilué à la concentration préconisée par le fournisseur (conjugué RACH FITC, Nordic), puis ajouté à raison de 50 µl par cupule contenant un virus de poulet. Un conjugué fluorescent anti-canard du commerce a été dilué à la concentration préconisée par le fournisseur (conjugué RADU FITC, Nordic), puis ajouté à raison de 50 µl par cupule contenant un virus de canard.

Les plaques ont été incubées 30 minutes à 38°C, puis le conjugué a été éliminé et les plaques rincées 3 fois avec de l'eau physiologique et 3 fois avec de l'eau déminéralisée.

Les plaques ainsi obtenues ont été observées au microscope à fluorescence. Une fluorescence intra-cytoplasmique intense a été observée avec le sérum spécifique du pneumovirus aviaire de la souche Colorado, tandis qu'aucune réaction n'a été observée avec les autres sérums.

### Exemple 4 Microscopie électronique

Des cultures de CEP (exemple 1) ou de cellules Véro (exemple 2) ont été inoculées avec le virus. Lorsque l'ECP est devenu visible, les tapis cellulaires ont été préfixés au glutaraldéhyde, ont subi une fixation osmique et ont été déshydratés à l'alcool éthylique.

Les zones d'intérêt présentant un début d'ECP ont ensuite été inclues en résine époxy et des coupes ultrafines des échantillons ont été réalisées et contrastées.

Les observations ont été réalisées au microscope électronique à transmission. On a observé la pénétration des virus par fusion de l'enveloppe virale avec la membrane cellulaire, l'accumulation intra-cytoplasmique de composés nucléoprotéiques et le bourgeonnement des particules virales au niveau de la membrane cytoplasmique. Des particules virales matures enveloppées polymorphes ont été observées dans l'espace extracellulaire. Certaines particules sont arrondies et d'un diamètre égal à 130 nm au minimum. D'autres particules sont filamenteuses avec une taille supérieure à 1000 nm. Des rendements présentant parfois des spicules ont été également observés. Ces images sont caractéristiques de la structure et de la morphogénèse des *Paramyxoviridae.*

### Exemple 5 Production du virus sur cellules Véro

Après décongélation, le virus provenant du stock décrit à l'exemple 2 peut être cultivé en flacons roulants.

Des cellules Véro ont été préparées en milieu MEM, pH 6,9 à 7,1, additionné de 5% de SVF. Le virus a été mélangé aux cellules avec une multiplicité d'infection (MOI) de 1 à 0,01 et le mélange a été réparti en flacons roulants à raison de 120×10⁶ cellules par flacon roulant. Les flacons ont été incubés à 38°C. Lorsque l'ECP causé par le virus fut optimum (plus de 50% du tapis cellulaire détruit), la suspension virale a été récoltée après agitation vigoureuse des flacons.

La récolte peut être mélangée à 50% avec un stabilisateur, par exemple une solution tamponnée. La suspension virale peut être conservée à une température inférieure ou égale à -40°C avant son utilisation ultérieure.

### Exemple 6 : Titrage du virus sur cellules Véro

Le titrage a été réalisé en microplaques 96 cupules en milieu MEM, pH 6,9 à 7,1, additionné de 3% de SVF. Des dilutions avec une raison d'ordre 10 de virus ont été mélangées à une suspension de cellules Véro dans la microplaque à raison de 0,1 ml de dilution de virus pour 1,8x10⁴ cellules sous 0,15 ml par cupule. Après de 6 à 9 jours d'incubation à 38°C avec 5% de CO₂, les cupules présentant un ECP généralisé ont été comptabilisées et le titre a été calculé en doses infectieuses pour culture de cellules 50 % (DICC50) par la méthode de Kärber.

### Exemple 7 : Préparation d'un vaccin vivant

Après décongélation du virus obtenu à l'exemple 5, la quantité de virus a été ajustée en fonction du titre désiré.

La suspension virale a été additionnée d'un stabilisateur SPGA (saccharose, phosphate, glutamate et albumine, EP-B1-0008255), répartie en flacons et lyophilisée. Le virus vaccinal a été titré comme à l'exemple 6. Le titre du vaccin a été ajusté à 10², 10³ et 10⁴ DICC50 par dose.

Avant son administration, le virus vaccinal peut être repris dans un diluant adjuvé contenant de l'hydroxyde d'alumine. L'adjuvant est composé d'un tampon phosphate isotonique contenant 2,1 grammes d'hydroxyde d'alumine par litre. Le vaccin lyophilisé a été repris dans le diluant de façon que 0,5 ml de diluant contienne une dose de virus vaccinal.

### Exemple 8 : Préparation d'un vaccin inactivé

Le virus a été cultivé selon la méthode décrite à l'exemple 5. La suspension virale a été clarifiée par centrifugation. Le virus a été inactivé par le formaldéhyde.

Une solution de formaldéhyde à 40% a été ajoutée de façon à obtenir une concentration finale de formaldéhyde de 0,1% dans la suspension virale. Le mélange a été maintenu à 37°C pendant 12h sous agitation modérée, puis refroidi à 5°C.

La suspension virale obtenue peut être concentrée par ultrafiltration 10 à 50 fois. La suspension virale inactivée concentrée ou non peut être conservée à une température de 5°C avant son utilisation.

On a réalisé une émulsion eau-dans-huile en ajoutant lentement en 15 min la phase aqueuse sur la phase huileuse sous agitation modérée à l'aide d'une turbine. On a homogénéisé pendant 10 min à 15 à 25 m/sec, puis l'émulsion ainsi formée a été refroidie à 5°C et stockée à cette température.

La phase aqueuse contient le virus inactivé et 3,2% v/v de polysorbate 80 (Tween® 80).

La phase huileuse contient 10% v/v de monooléate de sorbitan (Span® 80) et 90% v/v d'huile minérale (Drakeol® 6-VR).

Le ratio phase aqueuse sur phase huileuse est de 1:4.

Les suspensions virales pour le vaccin inactivé comportent 10^{6,2}, 10^{7,2} et 10^{8,2} DICC50 par ml.

### Exemple 9 :Méthode et programme de vaccination

Chez le jeune canard, le vaccin vivant adjuvé préparé comme décrit dans l'exemple 7 est administré par voie sous-cutanée à raison d'une dose de 0,5 ml dans la première semaine de vie et d'un rappel d'une dose de 0,5 ml entre 2 et 4 semaines après la première administration.

Chez les canards reproducteurs, le programme ci-dessus est appliqué, puis un rappel avec le vaccin vivant est effectué vers la 10ème semaine d'âge. Un rappel avec une dose de 0,3 ml de vaccin inactivé huileux (exemple 8) est ensuite pratiqué avant chaque période de ponte.

### Exemple 10 : Méthode de diagnostic

Le diagnostic de l'infection est réalisé par isolement viral selon la méthode décrite à l'exemple 1 ou par sérologie par une méthode de séroneutralisation.

La séroneutralisation est réalisée sur culture de cellules Véro en microplaques de 96 cupules. Les sérums à tester sont dilués de quart en quart dans du milieu MEM, pH 6,9 à 7,1, additionné de 1 % de SVF, directement dans les plaques de microtitration. On utilise une cupule par sérum et par dilution, à raison d'un volume final de 0,1 ml de sérum dilué par cupule. Le virus utilisé à la concentration de 200 DICC50 sous 0,025 ml est rajouté dans chaque cupule. Un temps de contact de 30 minutes à 38°C est réalisé. Les cellules VERO sont rajoutées dans chaque cupule à raison de 1.8X10⁴ cellules sous 0,15 ml de milieu MEM, pH 6,9 à 7,1, additionné de 1% de SVF. Un sérum négatif, un sérum positif témoin, ainsi que des témoins cellules sont mis en place pour chaque réaction. La lecture s'effectue après 9 jours d'incubation à 38°C avec 5% de CO₂, sous film plastique. Le pourcentage approximatif d'inhibition de l'ECP (0%, 50%, 100%) est apprécié. La dernière dilution entraînant une inhibition complète de l'ECP exprime le titre 100% du sérum testé. La dernière dilution entraînant une inhibition de 50% de l'ECP exprime le titre 50% du sérum testé.

## Revendications

1. Culture de pneumovirus aviaire dénommée C990427 et déposée auprès de la CNCM sous le numéro d'accès l-2353.

2. Préparation immunogène ou vaccin contre la pneumovirose aviaire, comprenant le pneumovirus aviaire C990427 dont un échantillon est déposé auprès de la CNCM sous le numéro d'accès l-2353, dans un véhicule ou excipient acceptable sur le plan vétérinaire, et éventuellement un adjuvant

3. Préparation immunogène ou vaccin selon la revendication 2, comprenant du virus vivant atténué comme antigène.

4. Préparation immunogène ou vaccin selon la revendication 3, comportant un adjuvant aqueux, de préférence de l'hydroxyde d'alumine.

5. Préparation immunogène ou vaccin selon la revendication 3 ou 4, comportant de 10² à 10⁶ DICC50 par doses, de préférence de 10² à 10⁴.

6. Préparation immunogène ou vaccin selon l'une des revendications 3 à 5, comportant en outre au moins un antigène provenant d'au moins un autre agent pathogène aviaire, de préférence choisi dans le groupe consistant en virus de la maladie Newcastle, virus de la bronchite infectieuse, virus de la maladie de Gumboro, parvovirus du canard, pneumovirus aviaires.

7. Préparation immunogène ou vaccin selon la revendication 2, comprenant du virus inactivé comme antigène.

8. Préparation immunogène ou vaccin se!on la revendication 7, comportant un adjuvant aqueux, de préférence l'hydroxyde d'alumine, ou formulé sous forme d'émulsion, de préférence une émulsion eau-dans-huile.

9. Préparation immunogène ou vaccin selon la revendication 8, formulé sous forme d'une émulsion eau-dans-huile contenant du polysorbate 80 dans la phase aqueuse, et de l'huile minérale et du monooléate de sorbitan dans la phase huileuse.

10. Préparation immunogène ou vaccin selon l'une des revendications 7 à 9, comportant de 10³ à 10⁸ DICC50 par doses, de préférence de 10⁵ à 10⁷, avant inactivation.

11. Préparation immunogène ou vaccin selon l'une des revendications 7 à 10, comportant en outre au moins un antigène provenant d'un autre agent pathogène aviaire, de préférence choisi parmi le virus de la maladie de Newcastle, le virus de là bronchite infectieuse, le virus de la maladie de Gumboro, l'adénovirus du syndrome de chute de ponte, le parvovirus du canard, le paramyxovirus de type III, les pneumovirus aviaires.

12. Utilisation du pneumovirus C990427 déposé auprès de la CNCM sous le numéro d'accès I-2353, pour la réalisation d'une préparation immunogène ou d'un vaccin destiné à être administré à des oiseaux, en particulier volailles, tels que canards et gallinacés.

13. Utilisation d'un antigène ou immunogène du pneumovirus C990427 déposé auprès de la CNCM sous le numéro d'accès I-2353, pour la réalisation d'une préparation immunogène ou d'un vaccin destiné à être administré à des oiseaux, en particulier volailles, tels que canards et gallinacés.

14. Utilisation selon la revendication 12 ou 13, destinée à une administration chez le canard.

15. Préparation immunogène ou vaccin contre la pneumovirose aviaire, comprenant un antigène spécifique du pneumovirus aviaire C990427 dont un échantillon est déposé auprès de la CNCM sous le numéro d'accès l-2353, dans un véhicule ou excipient acceptable sur le plan vétérinaire, et éventuellement un adjuvant.

## Patentansprüche

1. Kultur des als C990427 bezeichneten und bei der CNCM unter der Zugangsnummer I-2353 hinterlegten Vogel-Pneumovirus.

2. Immunogene Zubereitung oder Vakzin gegen Vogel-Pneumovirose umfassend das Vogel-Pneumovirus C990427, wovon eine Probe bei der CNCM unter der Zugangsnummer I- 2353 hinterlegt ist, in einem auf dem Veterinärgebiet annehmbaren Träger oder Arzneimittelträger und gegebenenfalls einen Arzneimittelhilfsstoff.

3. Immunogene Zubereitung oder Vakzin gemäß Anspruch 2, **dadurch gekennzeichnet, daß** sie als Antigen abgeschwächtes Lebendvirus umfassen.

4. Immunogene Zubereitung oder Vakzin gemäß Anspruch 3, **dadurch gekennzeichnet, daß** sie einen wäßrigen Arzneimittelhilfsstoff, vorzugsweise Aluminiumhydroxid umfassen.

5. Immunogene Zubereitung oder Vakzin gemäß Anspruch 3 oder 4, **dadurch gekennzeichnet, daß** sie 10² bis 10⁶, vorzugsweise 10² bis 10⁴ DICC50 je Dosis umfassen.

6. Immunogene Zubereitung oder Vakzin gemäß einem der Ansprüche 3 bis 5, **dadurch gekennzeichnet, daß** sie außerdem mindestens ein Antigen umfassen, das aus mindestens einem anderen pathogenen Vogel-Agens stammt und vorzugsweise aus der Gruppe ausgewählt ist, die aus dem Virus der Newcastle-Krankheit, dem Virus der infektiösen Bronchitis, dem Virus der Gumboro-Krankheit, dem Enten-Parvovirus und dem Vogel-Pneumovirus besteht.

7. Immunogene Zubereitung oder Vakzin gemäß Anspruch 2, **dadurch gekennzeichnet, daß** sie als Antigen inaktiviertes Virus umfassen.

8. Immunogene Zubereitung oder Vakzin gemäß Anspruch 7, **dadurch gekennzeichnet, daß** sie einen wäßrigen Arzneimittelhitfsstoff, vorzugsweise Aluminuimhydroxid umfassen oder in Form einer Emulsion, vorzugsweise einer Wasser-in-öl-Emulsion formuliert sind.

9. Immunogene Zubereitung oder Vakzin gemäß Anspruch 8, **dadurch gekennzeichnet, daß** sie in Form einer Wasser-in-Öl-Emulsion formuliert sind, die Polysorbat 80 in der wäßrigen Phase und Mineralöl und Sorbitanmonooleat in der Ölphase enthält.

10. Immunogene Zubereitung oder Vakzin gemäß einem der Ansprüche 7 bis 9, **dadurch gekennzeichnet, daß** sie vor der Inaktivierung 10³ bis 10⁸, vorzugsweise 10⁵ bis 10⁷ DICC50 je Dosis umfassen.

11. Immunogene Zubereitung oder Vakzin gemäß einem der Ansprüche 7 bis 10, **dadurch gekennzeichnet, daß** sie außerdem mindestens ein Antigen umfassen, das aus einem anderen pathogenen Vogel-Agens stammt und vorzugsweise aus der Gruppe ausgewählt ist, die aus dem Virus der Newcastle-Krankheit, dem Virus der infektiösen Bronchitis, dem Virus der Gumboro-Krankheit, dem Adenovirus des Syndroms der verminderten Legeleistung, dem Enten-Parvovirus, dem Paramyxovirus Typ III und dem Vogel-Pneumovirus besteht.

12. Verwendung des bei der CNCM unter der Zugangsnummer 1-2353 hinterlegten Pneumovirus C990427 zur Herstellung einer immunogenen Zubereitung oder eines Vakzins, die zum Verabfolgen an Vögel, insbesondere Geflügel wie etwa Enten und Hühner bestimmt sind.

13. Verwendung eines Antigens oder Immunogens des bei der CNCM unter der Zugangsnummer 1-2353 hintertegten Pneumovirus C990427 zur Herstellung einer immunogenen Zubereitung oder eines Vakzins, die zum Verabfolgen an Vögel, insbesondere Geflügel wie etwa Enten und Hühner bestimmt sind.

14. Verwendung gemäß Anspruch 12 oder 13, die zur Verabfolgung an Enten bestimmt ist.

15. Immunogene Zubereitung oder Vakzin gegen Vogel-Pneumovirose, die ein für das Vogel-Pneumovirus C990427, wovon eine Probe bei der CNCM unter der Zugangsnummer 1-2353 hinterlegt ist, spezifisches Antigen in einem auf dem Veterinärgebiet annehmbaren Träger oder Arzneimittelträger und gegebenenfalls einen Arzneimittelhilfsstoff umfaßt.

## Claims

1. An avian pneumovirus culture designated C990427 and deposited at the CNCM under the access number I-2353.

2. An immunogenic preparation or vaccine against avian pneumovirosis comprising the avian pneumovirus C990427, a sample of which is deposited at the CNCM under the access number I-2353, in a vehicle or excipient which is acceptable from the veterinary point of view and optionally an adjuvant.

3. An immunogenic preparation or vaccine according to claim 2 comprising live attenuated virus as an antigen.

4. An immunogenic preparation or vaccine according to claim 3 comprising an aqueous adjuvant, preferably aluminium, hydroxide.

5. An immunogenic preparation or vaccine according to claim 3 or claim 4 comprising from 10² to 10⁶ CCID50 per doses, preferably from 10² to 10⁴.

6. An immunogenic preparation or vaccine according to one of claims 3 to 5 further comprising at least one antigen originating from at least one other avian pathogenic agent, preferably selected from the group consisting of Newcastle disease virus, infectious bronchitis virus, Gumboro disease virus, duck parvovirus and avian pneumoviruses.

7. An immunogenic preparation or vaccine according to claim 2 comprising inactivated virus as an antigen.

8. An immunogenic preparation or vaccine according to claim 7 comprising an aqueous adjuvant, preferably aluminium hydroxide, or formulated in the form of an emulsion, preferably a water-in-oil emulsion.

9. An immunogenic preparation or vaccine according to claim 8 formulated in the form of a water-in-oil emulsion containing polysorbate 80 in the aqueous phase and mineral oil and sorbitan monooleate in the oily phase.

10. An immunogenic preparation or vaccine according to one of claims 7 to 9 comprising from 10³ to 10⁸ CCID50 per doses. preferably from 10⁵ to 10⁷ before inactivation.

11. An immunogenic preparation or vaccine according to one of claims 7 to 10 further comprising at least one antigen originating from at least one other avian pathogenic agent, preferably selected from Newcastle disease virus, infectious bronchitis virus, Gumboro disease virus, egg drop syndrome adenovirus. duck parvovinis, paramyxovirus type III and avian pneumoviruses.

12. Use of the pneumovirus C990427 deposited at the CNCM under the access number I-2353 for the production of an immunogenic preparation or a vaccine intended to be administered to birds, in particular poultry such as ducks. and gallinaceans.

13. Use of an antigen or immunogen of the pneumovirus C990427 deposited at the CNCM under the access number I-2353 for the production of an immunogenic preparation or a vaccine intended to be administered to birds, in particular poultry such as ducks and gallinaceans.

14. Use according to claim 12 or claim 13 intended for administration to ducks.

15. An immunogenic preparation or vaccine against avian pneumoviroses comprising an antigen specific for the avian pneumovirus C990427, a sample of which is deposited at the CNCM under the access number I-2353, in a vehicle or excipient which is acceptable from the veterinary point of view and optionally an adjuvant.
